# EUROPEAN PATENT SPECIFICATION

(11) **EP 0 538 314 B1**
(45) Date of publication and mention of the grant of the patent: **18.06.1997**
(21) Application number: 91912538.5
(22) Date of filing: 25.06.1991
(51) Int. Cl.: C07D 209/70, A61K 31/40

(54) **INDANO PYRROLIDINE CARBAMATES**
CARBAMATE DES INDAN-PYRROLIDINS
CARBAMATES D'INDIANO-PYRROLIDINE

(30) Priority: 12.07.1990 US 551970
(43) Date of publication of application: 28.04.1993
(73) Proprietor: PFIZER INC., New York, N.Y. 10017-5755 (US)
(72) Inventor: CHEN, Yuhpyng, L., Waterford, CT 06385 (US)
(74) Representative: Moore, James William, Dr.
(86) International application number: US9104320
(87) International publication number: WO9200961

(56) References cited:
- EP-A- 0 253 372
- GB-B- 1 351 754
- US-A- 3 703 529

## Description

### Background of the Invention

The present invention relates to indano-pyrrolidine carbamates. The compounds of the present invention inhibit brain acetylcholinesterase and are useful in the treatment of Alzheimer's disease. Moreover, intermediates of the compounds are useful as analgesic agents.

The increasing average age in the United States and most other developed nations has given rise to an alarming upswing in the incidence of Alzheimer's disease. Among the compounds being considered for use in treating Alzheimer's disease are physostigmine and derivatives thereof. The preparation and use of such compounds is referred to in M. Brufani et al., Eur. J. Biochem., 157, 115-120 (1986); Q.-S. Yu and A. Brossi, Heterocycles, 27, 745-750 (1988); European Patent Application Publication Number 0154864; European Patent Application Publication Number 0253372; K.L. Davis and R.C. Mohs, Am. J. Psychiatry, 139, 1421-1424 (1982); J.R. Atack et al., The Journal of Pharmacology and Experimental Therapeutics, 249, 194-202 (1989); R.G Longnecker, Spectrum: Pharmaceuticals Products and Technologies, April 1988, pages 1-39 to 1-45; and United States Patent Application No. 166,824, filed March 4, 1988, published by the National Technical Information Service.

US-A-3,703,529 discloses certain indeno[2,1-b]pyrrole derivatives which are useful as analgesic or antiinflammatory agents, whilst a series of structurally related indeno[2,1-b]pyrroles having analgesic activity and useful for the relief of pain and as antitussives is disclosed in GB-B-1,351,754. In EP-A-0253372 a group of pyrrolo[2,3-b]indoles, useful as memory-enhancing and analgesic agents, is described.

### Summary of the Invention

The present invention relates to compounds of the formula I 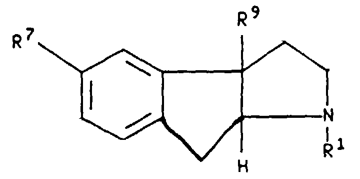 wherein
R¹ is (C₁ to C₈)alkyl or phenyl (C₁ to C₄)alkyl;
R⁷ is OCONR¹³H or OCSNR¹³H wherein R¹³ is (C₁ to C₁₂)alkyl, phenyl or phenyl (C₁-C₈)alkyl; and
R⁹ is hydrogen or (C₁ to C₈)alkyl;
and pharmaceutically acceptable acid addition salts thereof.

As used herein and unless indicated otherwise, the term alkyl includes straight chain and branched groups as well as alkyl groups having combinations of straight chain and branched portions.

The present invention also relates to pharmaceutical composition for the treatment of Alzheimer's disease comprising a compound of the formula I and to the use of compounds of the formula I in treating Alzheimer's disease. The present invention also relates to intermediates of the compounds of the formula I.

Preferred are those compounds wherein R¹ is methyl, ethyl, propyl, benzyl; R⁷ is OCONR¹³H where R¹³ is (C₁-C₁₂) alkyl, benzyl, phenyl; and R⁹ is methyl or hydrogen.

Preferred are those compounds wherein R¹ is methyl, R⁹ is methyl and R⁷ is OCONR¹³H with R¹³ C₁-C₇ alkyl.

Also preferred are those compounds wherein R¹ is ethyl, R⁹ is methyl and R⁷ is OCONR¹³H with R¹³ C₁-C₇ alkyl.

Preferred also are those compounds wherein R¹ is propyl R⁹ is methyl and R⁷ is as previously defined.

Preferred are those compound wherein R¹ is benzyl, R⁹ is methyl and R⁷ is as previously defined.

Specific preferred compounds of the present invention are the following:
(+/-)-1,2,3,3a,8,8a-hexahydro-1-ethyl-3a-methyl-indeno[2,1-b]pyrrol-5-ol, heptyl carbamate ester;
(-)-1,2,3,3a,8,8a-hexahydro-1-ethyl-3a-methyl-indeno[2,1-b]pyrrol-5-ol, heptyl carbamate ester;
(+)-1,2,3,3a,8,8a-hexahydro-1-ethyl-3a-methyl-indeno[2,1-b]pyrrol-5-ol, n-heptyl carbamate ester;
(+/-)-1,2,3,3a,8,8a-hexahydro-1-ethyl-3a-methyl-indeno[2,1-b]pyrrol-5-ol, n-hexyl carbamate ester;
(-)-1,2,3,3a,8,8a-hexahydro-1-ethyl-3a-methyl-indeno[2,1-b]pyrrol-5-ol, n-hexyl carbamate ester;
(+)-1,2,3,3a,8,8a-hexahydro-1-ethyl-3a-methyl-indeno[2,1,b]pyrrol-5-ol, n-hexyl carbamate ester;
(+/-)-1,2,3,3a,8,8a-hexahydro-1-ethyl-3a-methyl-indeno[2-1-b]pyrrol-5-ol, n-butyl carbamate ester;
(+/-)-1,2,3,3a,8,8a-hexahydro-1-ethyl-3a-methyl-indeno[2,1-b]pyrrol-5-ol, n-propyl carbamate ester;
(+/-)-1,2,3,3a,8,8a-hexahydro-1-ethyl-3a-methyl-indeno[2,1-b]pyrrol-5-ol, methyl carbamate ester;
(+/-)-1,2,3,3a,8,8a-hexahydro-1-ethyl-3a-methyl-indeno[2,1-b]pyrrol-5-ol, phenyl carbamate ester;
(+/-)-1,2,3,3a,8,8a-hexahydro-1-ethyl-3a-methyl-indeno[2,1-b]pyrrol-5-ol, benzyl carbamate ester;
(+/-)-1,2,3,3a,8,8a-hexahydro-1-ethyl-3a-methyl-indeno[2,1-b]pyrrol-5-ol, (S)- methylbenzyl carbamate ester;
(+/-)-1,2,3,3a,8,8a-hexahydro-1,3a-dimethyl-indeno-[2,1-b]pyrrol-5-ol, n-heptyl carbamate ester tosylate salt;
(+/-)-1,2,3,3a,8,8a-hexahydro-1,3a-dimethyl-indeno[2,1-b]pyrrol-5-ol, n-hexyl carbamate ester;
(+/-)-1,2,3,3a,8,8a-hexahydro-1-n-propyl-3a-methyl-indeno[2,1-b]pyrrol-5-ol, n-heptyl carbamate ester;
(+/-)-1,2,3,3a,8,8a-hexahydro-1-benzyl-3a-methyl-indeno[2,1-b]pyrrol-5-ol, methyl carbamate ester;
(+/-)-1,2,3,3a,8,8a-hexahydro-1-benzyl-3a-methyl-indeno[2,1-b]pyrrol-5-ol, n-heptyl carbamate ester;
[+/-]-1,2,3,3a,8,8a-hexahydro-1-propyl-indeno-[2,1-b]pyrrol-5-ol, n-heptyl carbamate ester;
[+/-]-1,2,3,3a,8,8a-hexahydro-1-propyl-indeno-[2,1-b]pyrrol-5-ol, n-hexyl carbamate ester;
[+/-]-1,2,3,3a,8,8a-hexahydro-1-methyl-indeno-[2,1-b]pyrrol-5-ol, heptyl carbamate ester;
[+/-]-1,2,3,3a,8,8a-hexahydro-1-methyl-indeno-[2,1-b]pyrrol-5-ol, hexyl carbamate ester;
(+/-)-1,2,3,3a,8,8a-hexahydro-1-ethyl-indeno-[2,1-b]pyrrol-5-ol, heptyl carbamate ester;
(+/-)-1,2,3,3a,8,8a-hexahydro-1-ethyl-indeno-[2,1-b]pyrrol-5-ol, propyl carbamate ester;
(+/-)-1,2,3,3a,8,8a-hexahydro-1-n-propyl-3a-methyl-indeno[2,1,b]pyrrol-5-ol; n-hexyl carbamate esters;
(-)-1,2,3,3a,8,8a-hexahydro-1-propyl-3a-methyl-indeno-[2,1,b]-pyrrol-5-ol, n-heptyl carbamate ester;
(+/-)-1,2,3,3a,8,8a-hexahydro-1-ethyl-indeno[2,1-b]-pyrrol-5-ol, butyl carbamate ester;
(+/-)-1,2,3,3a,8,8a-hexahydro-1-ethyl-indeno[2,1-b]-pyrrol-5-ol, hexyl carbamate ester;

Preferred compositions of the present invention contain the foregoing preferred compounds. More preferred compositions of the present invention contain the foregoing more preferred compounds and specific preferred compounds.

The present invention is also directed to a compound of the formula: 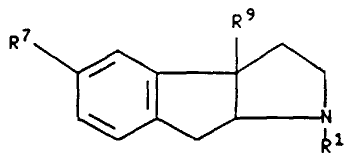 wherein
R¹ is (C₁ to C₈)alkyl or phenyl (C₁ to C₄)alkyl;
R⁷ is O(C₁ to C₄)alkyl; and
R⁹ is (C₁ to C₄)alkyl;
provided that when R⁷ is methoxy and R⁹ is methyl, then R¹ is not hydrogen, methyl, n-propyl, n-pentyl, benzyl or 2-phenylethyl; and pharmaceutically acceptable acid addition salts thereof.

The present invention is also directed to a compound of the formula: 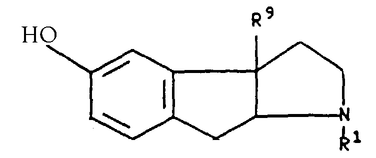 wherein
R¹ is (C₁ to C₈)alkyl or phenyl (C₁ to C₄)alkyl; and
R⁹ is (C₁ to C₄)alkyl;
provided that when R⁹ is methyl, then R¹ is not hydrogen or methyl; and pharmaceutically acceptable acid addition salts thereof.

### Detailed Description of the Invention

Compounds of the present invention may be prepared as described below.

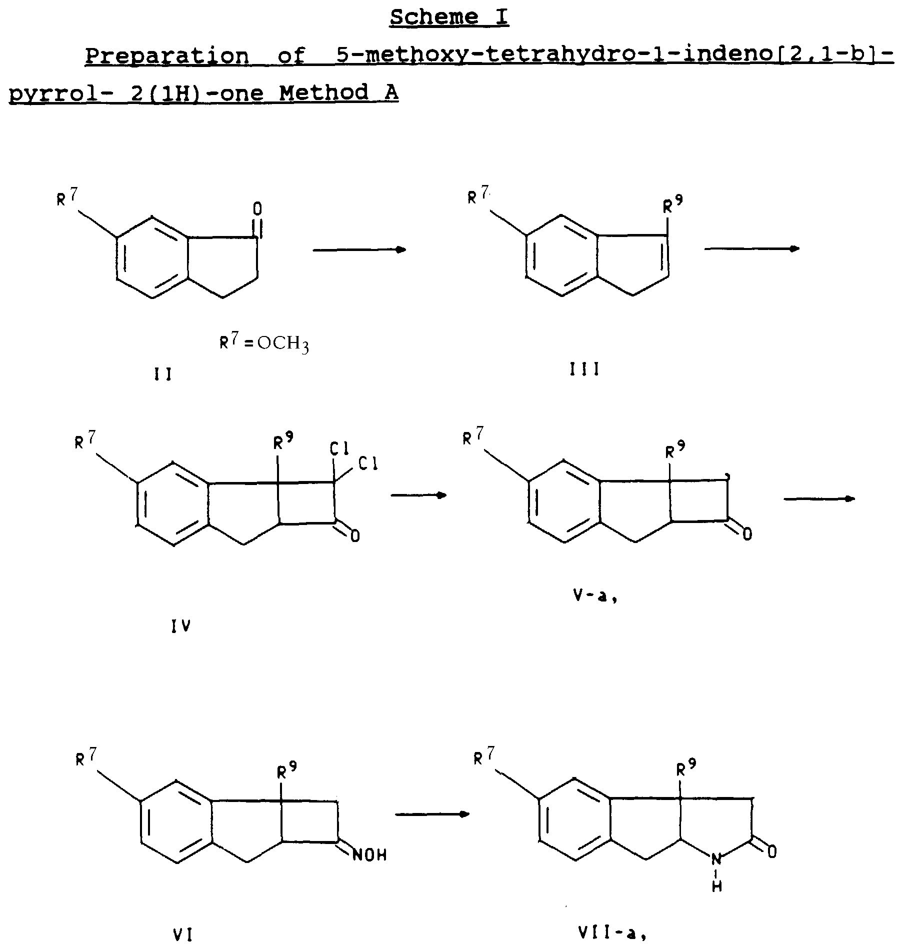

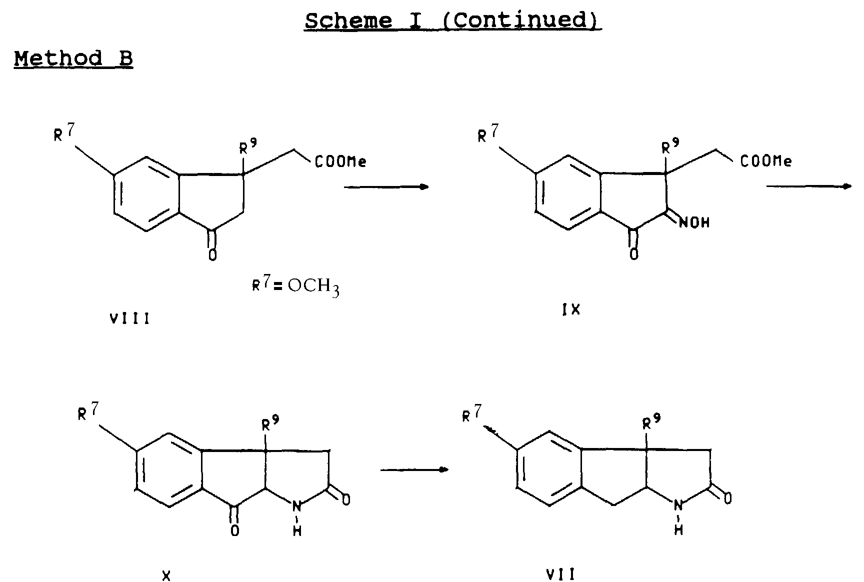

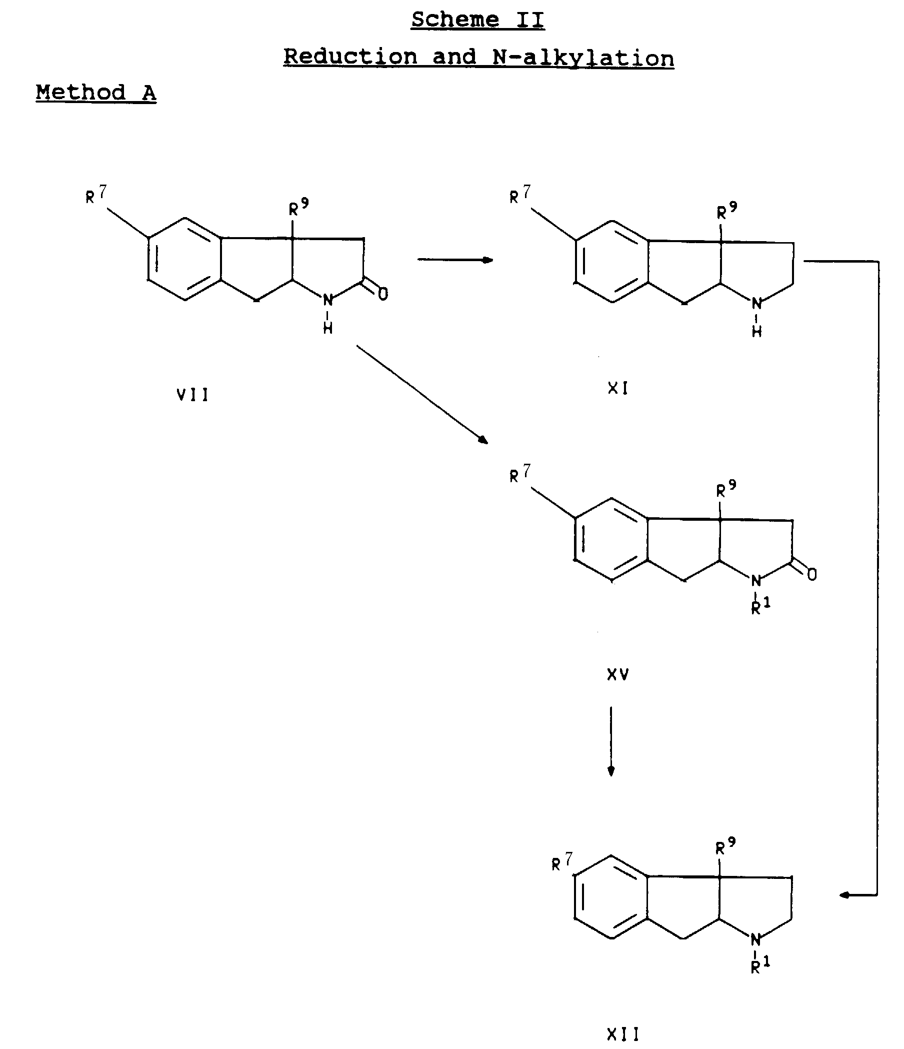

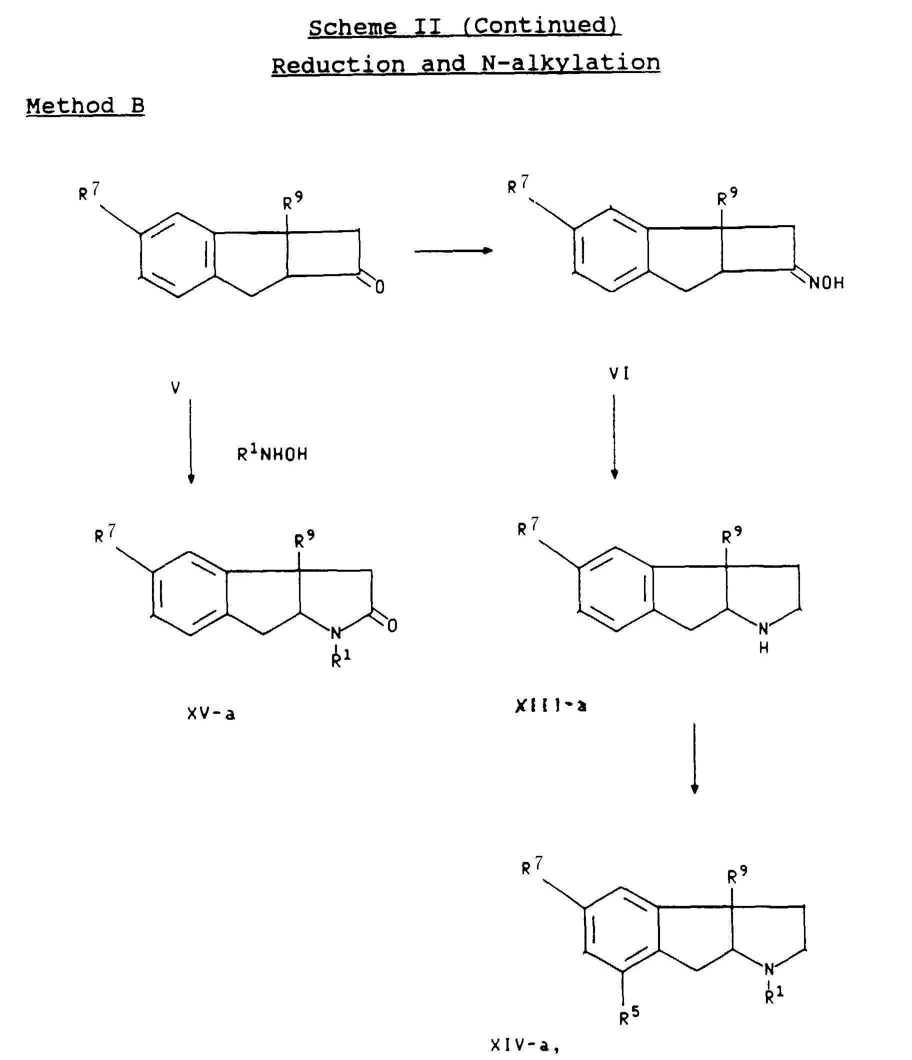

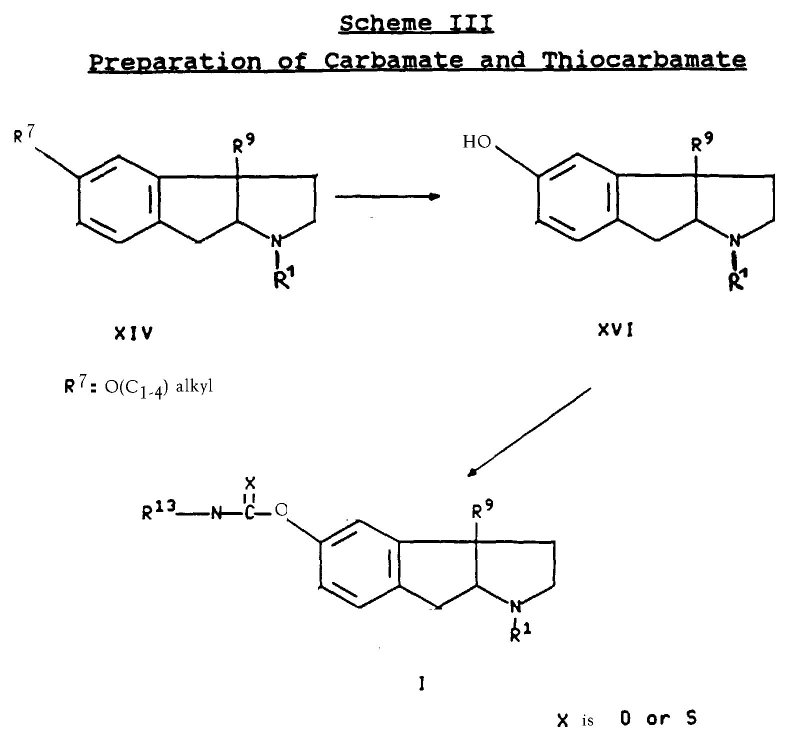

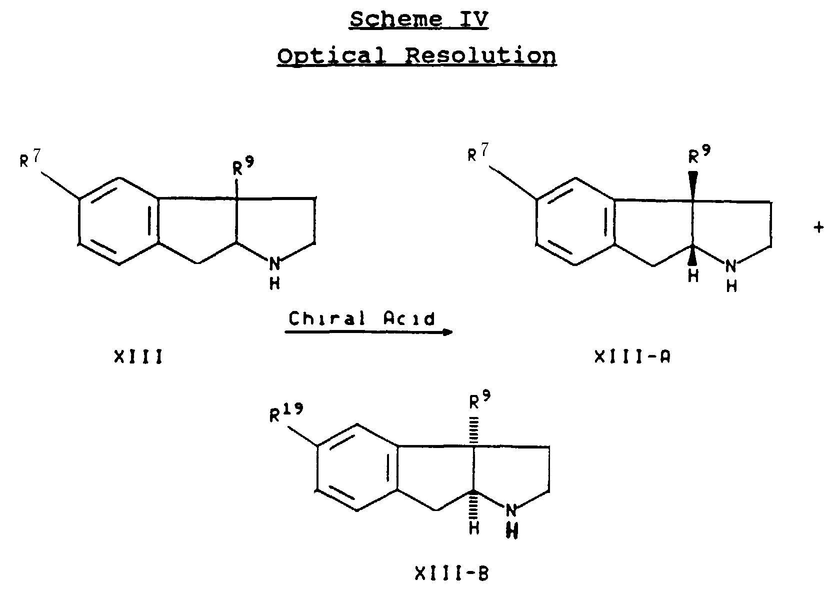

### Method B

XIV or I was resolved with chiral acid

### Method C

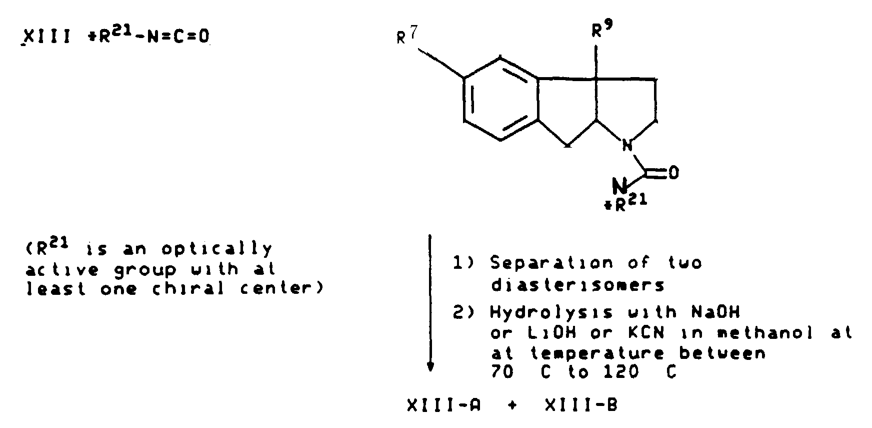

### Scheme I:

Method A
   a) In the first step, a compound of formula II is reacted with an organometallic (i.e. methyl magnesium bromide) In a reaction inert solvent (THF) followed by quenching with a suitable acid (HCl) to yield compound III.
   b) A compound of the formula III then undergoes [2+2] cycloaddition with a suitable ketene i.e. dichloro- ketene, to afford a compound of formula IV.
   c) Reduction of IV with Zn yields compound V-a.
   d) Beckmann rearrangement of VI yields formula VII-a.
Method B
   a) Formula X can be prepared via an oxime formation [(C₁ to C₆)alkyl nitrite in an aprotic solvent, preferably ether, THF, in the presence of gaseous HCl] followed by hydrogenation [in the presence of Pd/C at 1-6 atm in acid, preferably Acetic acid/gaseous HCl or in alcohol (e.g., ethanol) in the presence of gaseous HCl starting from compound VIII.
   b) formula VII can be obtained by hydrogenation (Pd/C in Acetic acid 3-6 atm at 50-100°C, preferably at 3 atm at 70°C) of formula X.

### Scheme II

Method A
   a) Compound XI can be prepared from compound VII by reduction (lithium aluminum hydride in THF or ether at temperature between 20°C to 100°C, preferably at 50-70°C; or borane-tetrahydrofuran complex in THF at a temperature between 20°C to 100°C, preferably at 50-70°C).
   b) Compound XII can be prepared from formula XI by acylation with acylating agents (such as acetic anhydride, acetyl chloride, propionyl chloride, benzolyl chloride in the presence of a base such as pyridine or triethylamine in an inert solvent such as tetrahydrofuran, methylene chloride or chloroform at a temperature from about 20°C to 100°C), followed by reduction with lithium aluminum hydride or borane-methyl sulfide complex in an inert solvent, preferably in THF or ether at 20°C to 100°C, preferably at 70°C. Compound XII (R¹=Me) can also be prepared by hydrogenation (Pd/C in alcohol at 1-5 atm at 20°C) in the presence of 37% formaldehyde in alcohol, preferably ethanol or methanol.
   c) In an alternative method, compounds of formula XII can be prepared by alkylation of VII in the presence of a base such as sodium hydride, sodium alkoxide, followed by reduction in a similar manner to that described above.
Method B
   a) XIII-a can be prepared directly from VI by reacting with diisobutylaluminum hydride according to the procedure set forth in (Tetrahedron Lett., 24, 4711, 1983).
   b) XV-a (R¹ is a group other than hydrogen) can be prepared by the nitrone rearrangement from V and N-alkyl, N-benzyl or N-phenyl hydroxy amine according to the method analogous to that set forth in (J. Org. Chem., 47, 3876, 1982; Tetrahedron, 44, 5209, 1988).
   c) Formula XIV-a can be prepared from formula XIII-a in a similar manner to that described for the transformation of XI to XII shown in Scheme II.

### Scheme III

a) Formula XVI can be prepared by hydrolysis of formula XIV with 48% HBr (at 20°C to refluxing temperature, preferably at refluxing temperature), or a Lewis acid such as boron tribromide, aluminum trichloride, or trimethylsilyl iodide in an aprotic solvent such as methylene chloride or chloroform at a temperature between -78°C to 50°C, preferably at -40°C to 20°C.
b) Compound I can be prepared by reacting a compound of formula XVI with R¹³N=C=O or R¹³N=C=S in the presence of a catalytic amount of base such as sodium hydride, sodium, or sodium alkoxide in an aprotic solvent such as tetrahydrofuran, ether, benzene, toluene at 0°C to 50°C, preferably at 20°C.
c) Compound I can also be prepared by treatment of XVI with 1,1'-carbonyldiimidazole or 1,1'-thiocarbonyldiimidazole and R¹³NH₂ in an aprotic solvent such as methylene chloride, tetrahydrofuran, or toluene at -40°C to 100°C, preferably at 20°C to 70°C. In an alternative method compound I can be prepared by treatment of XVI with R¹³HNCXCl in the presence of base such as potassium carbonate or triethyl amine in an aprotic solvents such as dimethylformamide, methylene chloride, or tetrahydrofuran at temperature of from about 20°C to refluxing temperature.

### Scheme IV

a) Optical resolution can be performed at any amine stage such as intermediate XIII, XIV or final compound I with chiral acids such as mandelic acid, tartaric acid, di-p-toluoyl-tartaric acid as shown in Scheme IV.
b) The resolution can also performed by reacting XIII with a chiral alkyl isocyanate to form diastereomers, then separating the diastereomers by either recrystallization or chromatography, followed by hydrolysis to give each enantiomer. The optical resolution can also performed according to the methods analogous to those set forth in J. med. Chem. 29, 2268, 1986; Helv. Chim. Acta, 69, 283 and 1486, 1986.

Compounds of Formula I are capable of forming acid addition salts with pharmaceutically acceptable acids. The acid addition salts may be prepared by reacting the base form of the appropriate compound of formula I with one or more equivalents, preferably with 1 equivalent, of the appropriate acid in an organic solvent, for example, diethyl ether, an ethanol/diethyl ether mixture, ethanol, methanol, or i-propanol. Suitable acids to form these salts include the common mineral acids, e.g. hydrohalic, sulfuric or phosphoric acid; the organic acids, e.g. ascorbic, citric, lactic, aspartic, p-toluenesulfonic acid; methanesulfonic acid; di-p-toluoyl-(D) or (L)tartaric acid; mandelic acid; fumaric acid; succinic acid; salicylic acid; oxalic acid or tartaric acid or their aqueous solutions whose pH has been adjusted to 5.5 or less; and acids which are sparingly soluble in body fluids and which impart slow-release properties to their respective salts, e.g. pamoic or tannic acid or carboxymethyl cellulose. Preferred salts are the hydrochloride salt, mesylate salt, tartrate salt, or di-p-toluoyl-L-tartrate salt.

The compounds of formula I and the pharmaceutically acceptable salts thereof are useful in the treatment of various memory dysfunctions associated with decreased cholinergic function such as Alzheimer's Disease. Additionally, the compounds lead to stimulation of neuromuscular transmission, enhancement of excitation in excitable tissues (nerve, and smooth and striated muscle) as well as restoration of conductance in nerves and neuromuscular synapses in the case of injury thereof. The compounds of this invention are, in general, less toxic and have a broader therapeutic window than known compounds such as tacrine and physotigmine, making them therapeutically preferred.

In treating Alzheimer's disease, the dosage of the compounds of the present invention will vary with the form of administration and the particular compound chosen. Furthermore, it will vary with the particular subject as well as the age, weight and condition of the subject under treatment as well as with the nature and extent of the symptoms. Generally, however, a dose in the range of about 1 to about 300 mg/day, taken in single or divided doses, will be administered. The preferred dose is in the range of from about 1 to about 300 mg/day in single or divided doses.

Generally, treatment is initiated with small dosages substantially less than the optimum dose of the compound. Thereafter, the dosage is increased by small increments until the optimum effect under the circumstances is reached.

The compounds of the present invention are used alone or in combination with pharmacologically acceptable carriers, the proportion of which is determined by the solubility and chemical nature of the compound, chosen route of administration and standard medical practice. For example, they are administered orally in the form of capsules, tablets, suspensions or solutions or they may be injected parenterally. Capsules and tablets are the preferred mode of administration. For parenteral administration, they can be used in the form of a sterile solution containing other solutes, for example, enough saline or glucose to make the solution isotonic.

Capsule and tablet compositions may contain the active ingredient in admixture with one or more pharmaceutical excipients suitable for the manufacture of capsules and tablets. Suitable pharmaceutical excipients are, for example, starch, milk sugar, and certain types of clay. The tablets can be uncoated or they can be coated by known techniques so as to delay disintegration and absorption in the gastrointestinal tract and thereby provide a sustained action over a longer period.

Aqueous suspensions of the compounds of formula I contain the active ingredient in admixture with one or more pharmaceutical excipients suitable for the manufacture of aqueous suspensions. Suitable excipients are, for example, methylcellulose, sodium alginate, gum acacia, lecithin and so forth. Aqueous suspensions can also contain one or more preservatives, one or more coloring agents, one or more flavoring agents and one or more sweetening agents.

Non-aqueous suspensions can be formulated by suspending the active ingredient in a vegetable oil for example, arachis oil, olive oil, sesame oil, or coconut oil, or in mineral oil, for example liquid paraffin, and the suspension may contain a thickening agent, for example beeswax, hard paraffin or cetyl alcohol. These compositions can also contain a sweetening agent, flavoring agent and antioxidant.

The following examples illustrate the preparation and properties of the compounds of the present invention. All melting points are uncorrected. Thin layer chromatography (TLC) was conducted on silica gel.

### Preparations and Examples

### Preparation 1 5-Methoxy-3a-methyl-3,3a,8,8a-tetrahydro-1-indeno[2,1-b]pyrrol-2-(1H)-one

### A. 1-Methyl-6-Methoxy-1-indene

A solution of 6-methoxy-1-indanone (8.1 g, 50 mmol) in dry THF (tetrahydrofuran) was added dropwise to a solution of methyl magnesium bromide (3N in ether, 20 ml, 60 mmol) in dry THF at 0°C. After completion of addition, the mixture was stirred at room temperature (∼20°C) for 0.5 hour and quenched with aqueous HCl to pH 1 and extracted with ethyl acetate. The organic layer was washed with brine, dried, and concentrated to give 6.86 g of white crystals, mp 46-47°C; ¹H NMR (CDCl₃) δ 2.35 (s, 3H), 3.25 (m, 2H), 3.9 (s, 3H), 6.25 (m, 1H); 6.75 (dd, 1H), 6.9 (d, 1H), 7.35 (d, 1H) ppm; ¹³C NMR (CDCl₃) δ 13.0, 37.0, 55.6, 104.8, 110.3, 123.9, 130.2, 136.5, 139.8, 147.6, 158.95 ppm.

### B. 1-Methyl-6,7-(9-methoxy)benzo-2,2-dichloro[3,2,0]-hept- 3-one

To a stirred mixture of 1-methyl-6-methoxy-1- indene (2.632 g, 16.45 mmol) and activated Zinc (1.19 g, 18.23 mmol) in anhydrous ether was added a solution of Cl₃CCOCl (1.0 ml, 17 mmol) and POCl₃ (1.6 ml, 17 mmol) in ether. The mixture was refluxed for 2 hours, then stirred at room temperature overnight. Workup afforded a brown oil which can be used directly for the next dehalogenation step. 0.5 g material was purified through silica gel column purification to give white crystals, mp 65-67°C; ¹H NMR (CDCl₃) δ 1.8 (s, 3H), 3.0-3.3 (m, 2H), 3.8 (s, 3H), 3.96 (dd, 1H), 6.84 (m, 2H), 7.1 (m, 1H) ppm, Anal. (C₁₃H₁₂O₂Cl₂) C, H.

### C. 1-Methyl-6,7-(9-methoxy)benzo[3.2.0]hept-3-one

A solution of the crude material of the product of Preparation 1B in methanol was treated with 8 g of Zinc and 6.4 g of NH₄Cl and heated to 45-50°C overnight. The mixture was filtered through (diatomaceous earth (celite (trademark)) and the filtrate was concentrated to give a white solid which was purified through silica gel chromatography to give 2.423 g of white crystals, mp 71-72°C; ¹H NMR (CDCl₃) δ 1.23 (s, 3H), 2.95-3.35 (m, 4H), 3.55-3.65 (m, 1H), 3.8s, 3H), 6.7-6.8 (m, 2H), 7.05 (d, 1H) ppm; ¹³C NMR (CDCl₃) δ 24.5, 32.5, 44.1, 55.5, 61.0, 68.7, 108.9, 113.7, 125.6, 134.2, 149.5, 160 ppm; Anal. (C₁₃H₁₄O₂) C, H, N.

### D. 1-Methyl-6,7-(9-methoxy)benzo[3.2.0]hept-3-one oxime

A solution of the preparation of 1C (2.084 g, 10.3 mmol) in methanol was treated with NH₂OH, HCl and sodium acetate and stirred at room temperature overnight. Purification through silica gel column afforded 1.581 g of the title compound as white crystals, mp 116-117°C; ¹H NMR (CDCl₃) δ 1.55 (s, 3H), 2.9-3.3 (m, 4H), 3.4-3.6 (m, 1H), 3.78 (s, 3H), 6.65-6.8 (m, 2H), 7.05 (m, 1H), 8.3 (brs, 1H) ppm; ¹³C NMR (CDCl₃) δ 24.3, 35.1, 44.7, 48.0, 54.1, 55.5, 108.5, 113.5, 125.7, 184.6, 150.0, 159.5, 160.2 ppm, Anal. (C₁₃H₁5NO₂) C, H, N.

### E. 5-Methoxy-3a-methyl-3,3a,8,8a-tetrahydro-1-indeno[2,1-b]-pyrrol-2(1H)-one

A solution of the title compound of 1D Preparation (434 mg) in CH₂Cl₂ was treated with NaH (88 mg) at 0°C. After 5 minutes, the mixture was treated with tosyl chloride (419 mg) and stirred at 0°C for 8 hours and then at room temperature overnight. The mixture was quenched with 2N NaOH, extracted with CH₂Cl₂, dried and concentrated to dryness. The residue was triturated with ether to give 260 mg of a white solid. ¹H NMR (CDCl₃) δ 1.38 (s, 3H), 2.58 (ABq, 2H), 2.78 (d, 1H), 3.16 (dd, 1H), 3.77 (s, 3H) 4.03 (d, 1H), 6.51 (s, 1H, NH), 6.69 (s, 1H), 6.74 (d, 1H), 7.06 (d, 1H) ppm.

### Preparation 2

### A. 5-Methoxy-3,3a,8,8a,-tetrahydro-indeno[2,1-b]pyrrol-2-(1H)-one

### A. Methyl 5-methoxy-1-indanone-3-acetate

A solution of 5-methoxy-1-indanone-3-acetic acid (30.0 g, 0.136 mmol) in methanol (200 ml) was treated with concentrated H₂SO₄ (4 ml) and heated under reflux for 3 hours and then stirred at room temperature overnight. The resulting solution was concentrated to dryness. The residue was neutralized with saturated NaHCO3 and extracted with ether. The organic layer was washed with water, dried and concentrated to give the title compound as a yellow solid (29.5 g). ¹H NMR (DMSO-d₆) δ 2.48 (s, 2H), 3.18 (m, 2H), 3.46 (s, 3H), 3.86 (s, 3H), 4.24 (t, 1H), 7.0 (dd, J=1 and 7 Hz, 1H), 7.14 (d, J=1 Hz, 1H), 7.64 (d, J=1 and 7 Hz, 1H) ppm.

### B. 5-Methoxy-3-methoxycarbonylmethyl-1-indanone-2-one oxime

A solution of the title compound of Preparation 2A (29.15 g, 0.1244 mol) in anhydrous ether (200 ml) was saturated with HCl gas and cooled to 5-10°C in an ice bath and butyl nitrite was added dropwise and stirred for 3 hours. The product was filtered off to yield the title compound as an orange solid (28.8 g). ¹H NMR (DMSO-d₆) δ 2.46 (m, 2H), 3.16 (m, 2H), 3.42 (s, 3H), 3.82 (s, 3H), 4.2 (t, J=4Hz, 1H), 6.96 (dd, J=1 and 7 Hz, 1H), 7.12 (d, J=1Hz, 1H), 7.64 (d, J=7Hz, 1H) ppm.

### C. 5-Methoxy-3a,8a-dihydroindeno[2,1-b]pyrrol-2,8-(1H, 3H)-dione

A solution of the title compound of Preparation 2B (1.0 g, 3.8 mmol) in acetic acid (50 ml) saturated with HCl gas was treated with 10% Pd on charcoal (0.5 g) and hydrogenated at 50 psi for 1 hour. After removal of the catalyst, the filtrate was heated to reflux for 4 hours, and the reaction was monitored by thin layer chromatography (5% methanol in CHCl₃). Removal of solvents gave a brown solid. The solid was trituated with ether to give the title compound as a yellow solid (0.59 g). ¹H NMR (DMSO=d₆) δ 2.24 (dd, J=2 and 18Hz, 1H), 2.84 (dd, J=8 and 18Hz, 1H), 3.9 (s, 3H), 4.04 (m, 1h), 4.2 (d, J=4Hz, 1H), 7.04 (dd, J=1 and 7Hz, 1H), 7.18 (d, J=1Hz, 1H), 7.62 (d, J=7Hz, 1H), 8.58 (s, 1H, NH) ppm.

### D. 5-Methoxy-3,3a,8,8a-tetrahydro-indeno[2,1-b]pyrrol-2-(1H)-one

A solution of the title compound of Preparation 2°C (10 g, 0.046 mol) and 10% Pd in charcoal (5 g) in acetic acid (150 ml) was hydrogenated at 50 psi at 70°C for 4 hours. The catalyst was removed by filtration and the filtrate was evaporated to dryness to give a solid which was trituated with ether to give the title compound as a white solid (9.0 g). ¹H NMR (DMSO-d₆) δ 2.22 (d, J=14Hz, 1H), 2.64 (dd, J=10 and 14Hz, 1H), 2.72 (d, J=18Hz, 1H), 2.98 (dd, J=6 and 18Hz, 1H), 3.68 (s, 3H), 3.70 (m, 1H), 4.3 (t, J=6Hz, 1H), 6.72 (dd, J=2 and 8Hz, 1H), 6.8 (d, J=2Hz, 1H), 7.08 (d, J=8Hz, 1H) ppm.

### Preparation 3

### 5-Methoxy-3,3a,8,8a-tetrahydro-3a-methyl-indeno[2,1-b]pyrrol-2(1H)-one

Following the methods of Preparations 2A, 2B, 2C and 2D, the title compounds of Preparations 3A, 3B, 3C and 3D, respectively, were prepared.

### A. Methyl 5-methoxy-1-indanone-3-methyl-3-acetate

¹H NMR (CDCl₃) δ 1.4 (s, 3H), 2.52 (d, J=18z, 1H), 2.68 (ABq, J_{AB}=11 Hz, 2H), 2.97 (d, J=18 Hz, 1H), 3.56 (s, 3H), 3.83 (s, 3H), 6.8-6.9 (m, 2H), 7.6 (d, J=6 Hz, 1H) ppm.

### B. 5-Methoxy-3-methyl-3-methoxycarbonylmethyl-1-indanone-2-one oxime

¹H NMR (CDCl₃) δ 1.64 (s, 3H), 2.94 (d, J=17Hz, 1H), 3.4 (s, 3H), 3.87 (d, J=16Hz, 1H), 3.9 (s, 3H), 6.84 (d, J=2Hz, 1H), 6.92 (dd, J=2 and 8Hz, 1H), 7.82 (d, J=8Hz, 1H) ppm.

### C. 5-Methoxy-3a, 8a-dihydro-3a-methyl-indeno[2,1-b]pyrrol-2,8-(1H,3H)-dione

¹H NMR (CDCl₃) δ 1.61 (s, 3H), 2.60 (d, 2H), 3.82 (s, 1H), 3.90 (s, 3H), 6.40 (s, 1H, NH), 6.87 (d, J=2Hz, 1H), 6.95 (dd, J=2 and 8Hz, 1H), 7.68 (d, J=8Hz, 1H) ppm.

### D. 5-Methoxy-3,3a,8,8a-tetrahydro-3a-methyl-indeno[2,1-b]-pyrrol-2(1H)-one (4b)

Recrystallization from i-PrOH gave a white crystals, mp 197-198°C. ¹H NMR (CDCl₃) δ 1.38 (s, 3H), 2.58 (ABq, J_{AB}=7Hz, 2H), 2.78 (d, J=18Hz, 1H), 3.16 (dd, J=6 and 17Hz, 1H), 3.77 (s, 3H), 4.03 (d, J=6Hz, 1H), 6.51 (s, 1H, NH), 6.69 (s, 1H), 6.74 (d, J=8Hz, 1H), 7.06 (d, J=8Hz, 1H) ppm.

### Example 1

### 5-Methoxy-1,2,3,3a,8,8a-Hexahydro-indeno[2,1-b]pyrrole

To a suspension of LiAlH₄ (0.14 g, 3.69 mmol) in dry THF (10 ml) was added a solution of the title compound of Preparation 2D in THF (3 ml) dropwise. When the addition was complete, the mixture was heated to reflux for 5 hours. The mixture was cooled and treated by successive dropwise addition of H₂O (0.14 ml), 15% NaOH solution (0.42 ml), and H₂O (0.42 ml). The mixture was stirred for 20 minutes and granular precipitates was filtered off. The filtrate was dried and concentrated to yield the title compound as an oil 1.6 g. ¹H NMR (CDCl₃) δ 1.9 (m, 1H), 2.2 (m, 1H), 2.82 (d, J=15 Hz, 1H), 2.84 (m, 2H), 3.2 (dd, J=6 and 15 Hz, 1H), 3.7 (m, 1H), 3.98 (s, 3H), 4.0 (t, J=6Hz, 1H), 6.7 (m, 3H), 7.04 (d, J=7Hz, 1H) ppm.

### Example 2

### Resolution of racemic 5-methoxy-1,2,3,3a,8,8a-hexahydro-indeno[2,1-b]pyrrole

To a solution of racemic 1,2,3,3a,8,8a-hexahydro-5-methoxy-indeno[2,1-b]pyrrole (1.2 g, 6.34 mmol) in 2-propanol (5 ml) was added a solution of (S)-mandelic acid (0.96 g, 6.34 mmol) at room temperature and stirred for 1 hour. White precipitate formed and was filtered. The white solid was collected and washed with 2-propanol. The solid was basified to pH 13 and extracted with CH₂Cl₂. The organic layer was dried and concentrated to give 0.32 g of oil ([α]_{D}= -76.00°). The oil was converted to (S)-mandelic acid salt again, recrystallized, and basified to give 0.191 g of oil ([α]_{D}= -80.2°).

The filtrate was basified to give an oil, which was converted to (R)-mandelic acid salt, recrystallized from 2-propanol, basified to give 0.24 g of oil ([α]_{D}= +74.5°). This process was repeated several times to give 0.13 g of oil ([α]_{D}=+80.0°).

Both enantiomers were converted to the corresponding N₁-carbamate diasteroisomers by reaction with 1 eq. (S)-1-(phenyl)ethyl isocyanate in CH₂Cl₂ and evaporated to dryness. The optical purity was determined by ¹H NMR (CDCl₃). The methyl peak of the (-) isomer and (+) isomer showed at 1.45 ppm and 1.49 ppm, respectively.

### Example 3

### 5-methoxy-3a-methyl-1,2,3,3a,8,8a-hexahydro-indeno-[2,1-b]pyrrole

Following the method of Example 1, the title compound was prepared. ¹H NMR (CDCl₃) δ 1.3 (s, 3H), 1.8-2.15 (m, 2H), 2.6 (s, 1H, NH), 2.7-2.95 (m, 2H), 3.0 (m, 1H), 3.24 (dd, J=6 and 18Hz, 1H), 3.58 (dd, J=2 and 6Hz, 1H), 3.8 (s, 3H), 6.6-6.8 (m, 2H), 7.06 (d, J=7Hz, 1H) ppm.

### Example 4

### Resolution of racemic 5-methoxy-3a-methyl-1,2,3,3a,8,8a-hexahydro-indeno[2,1-b]pyrrole

The racemic form of the title compound was resolved with di-p-tol uyl-L-tartaric acid by recrystallizing the salts several times as described in Example 2, and basified to afford both pure optically active enantiomers as an oil with [α]_{D}= -97.3°, respectively.

### Example 5

### N-Alkylation of 5-Methoxy-1,2,3,3a,8,8a-hexahydroindeno[2,1-b]pyrrole and 3a methyl analogue

A solution of 5-methoxy-1,2,3,3a,8,8a-hexahydroindeno[2,1-b]pyrrole in CH₂Cl₂ was treated with 1.1 equivalents of alkanoyl chloride and (e.g., acetyl chloride, propionyl chloride and 1.1 equivalents of pyridine and stirred at room temperature for 1 hour. The mixture was washed with H₂O, extracted with CH₂Cl₂. The organic layer was washed with dilute HCl, then neutralized with saturated NaHCO₃, dried and concentrated to give the corresponding amide. To a solution of the amide in dry THF was added 3 equivalents of BH₃.DMS dropwise. After addition, the mixture was heated at reflux for 3 hours and cooled to 0°C and methanol was added. The resulting solution was treated with concentrated HCl and stirred at room temperature overnight. Solvent was evaporated and the residue was washed with saturated K₂CO₃ and H₂O, and extracted with CHCl₃. The organic layer was dried and concentrated to yield 1-alkylated product. Following this procedure, the following compounds were prepared:

### A. (+/-)-1,2,3,3a,8,8a-hexahydro-5-methoxy-1-ethylindeno[2,1-b]pyrrole:

¹H NMR (CDCl₃) δ 1.12 (t, 3H), 1.7-1.9 (m, 1H), 2.1-2.4 (m, 3H), 2.76-3.0 (m, 3H), 3.0-3.2 (m, 2H), 3.6-3.75 (m, 1H), 3.78 (d, 3H), 6.7 (m, 2H), 7.03 (m, 1H) ppm.

### B. (+/-)-1,2,3,3a,8,8a-Hexahydro-5-methoxy-1-propylindeno[2,1-b]pyrrole:

¹H NMR (CDCl₃) δ 0.92 (t, 3H), 1.4-1.9 (m, 4H), 2.15-2.4 (m, 2H), 2.6-3.3 (M, 5H), 3.5-3.8 (m, 4H), 6.7 (m, 2H), 7.05 (d, 1H) ppm.

### C. (+/-)-1,2,3,3a,8,8a-Hexahydro-5-methoxy-1-ethyl-3a-methyl-indeno[2,1-b]pyrrole:

¹H NMR (CDCl₃) δ 1.15 (t, 3H), 1.35 (s, 3H), 1.86-2.1 (m, 2H), 2.2-2.4 (m, 2H), 2.62-2.92 (m, 3H), 2.92-3.12 (m, 2H), 3.76 (s, 3H), 6.65 (d, J=2Hz, 1H), 6.68 (d, J=2Hz, 2H), 7.0 (d, J=7Hz, 1H) ppm.

### D. (-)-1,2,3,3a,8,8a-Hexahydro-5-methoxy-1-ethyl-3a-methyl-indeno[2,1-b]pyrrole:

[α]_{D} = -110.7°.

### E. (+/-)-1,2,3,3a,8,8a-Hexahydro-5-methoxy-1-n-propyl- 3a-methyl-indeno[2,1-b]pyrrole:

¹H NMR (CDCl₃) δ 0.9 (t, 3H), 1.3 (s, 3H), 1.4-1.6 (m, 2H), 1.8-2.4 (m, 4H), 2.6-3.1 (m, 5H), 3.76 (s, 3H), 6.66 (m, 2H), 7.02 (d, 1H) ppm.

### Example 6

### (+/-)-1,2,3,3a,8,8a-Hexahydro-5-methoxy-1-ethyl-3a-methyl-indeno[2,1-b]pyrrole:

A solution of the title compound of Example 3 (0.7 g) in acetic anhydride (25 ml) was heated at reflux for 1 hour and concentrated to dryness. The residue was neutralized with saturated NaHCO₃ and extracted with ethyl acetate to give a tan solid (0.78 g). The solid was dissolved in dry THF (25 ml) and treated with 2M BH₃.DMS in THF (4 ml, 8 mmol) at 0°C. The resulting solution was heated at reflux for 3 hours, then cooled to 0°C and methanol (10 ml) was added dropwise. The solvent was evaporated and 2N HCl (25 ml) was added and the mixture was heated at reflux for 3 hours and cooled to room temperature, neutralized with saturated K₂CO₃ and extracted with ethyl acetate, dried and concentrated to give the title compound as a colorless oil (0.73 g). ¹H NMR (CDCl₃) δ 1.15 (t, 3H), 1.35 (s, 3H), 1.86-2.1 (m, 2H), 2.2-2.4 (m, 2H), 2.62-2.92 (m, 3H), 2.92-3.12 (m, 2H), 3.76 (s, 3H), 6.65 (d, J=2Hz, 1H), 6.68 (d, J=2Hz, 2H), 7.0 (d, J=7Hz, 1H) ppm.

### Example 7

### 1,2,3,3a,8,8a-Hexahydro-5-methoxy-1-methyl-indeno-[2,1-b]pyrrole

A solution of 5-methoxy-1,2,3,3a,8,8a-hexahydroindeno[2,1-b]pyrrole (0.4 g, 2.11 mmol) in ethanol (30 ml) was treated with 37% formaldehyde in H₂O (0.35 ml) and 10% Pd in charcoal (0.2 g) and hydrogenated at 50 psi for 2.5 hours. The catalyst was removed by filtration through diatomaceous earth Celite (trademark) and the filtrate was concentrated and dried in vacuo to give the title compound as a solid (0.38 g). ¹H NMR (CDCl₃) δ 1.8-1.95 (m, 1H), 2.24-2.4 (m, 5H), 2.8-3.06 (m, 4H), 3.6-3.8 (m, 4H), 6.66 (m, 2H), 7.02 (m, 1H) ppm.

### Example 8

### 1,2,3,3a,8,8a-Hexahydro-5-methoxy-1,3a-dimethyl-indeno-[2,1-b]pyrrole

Following the method of Example 7, the title compound was prepared. ¹H NMR (CDCl₃) δ 1.3 (s, 3H), 1.9-2.15 (m, 2H), 2.36 (s, 3H), 2.35-2.5 (m, 1H), 2.5-2.7 (m, 1H), 2.7-3.05 (m, 3H), 3.75 (s, 3H), 4.7-4.9 (m, 1H), 6.6-6.7 (m, 2H), 7.0 (m, 1H) ppm.

### Example 9

### Preparation of Hexahydro-indeno[2,1-b]pyrrole carbamates

A solution of 5-methoxy-1-alkyl-1,2,3,3a,8,8a-hexahydroindeno[2,1-b]pyrrole in 48% HBr (7 ml/mmol) was heated at reflux for 4 hours and evaporated to dryness. The residue was dissolved in methanol and treated with 1 equivalent of sodium methoxide and stirred for 30 minutes. After removal of solvent, the residue was treated with a minimum amount of isopropanol (5 ml) and filtered to remove inorganic material. The filtrate was concentrated to dryness. The residue was treated three times with benzene and evaporated to dryness to give the corresponding 5-hydroxy compound. A solution of the 5-hydroxy derivative (e.g. (+/-)-1,2,3,3a,8,8a-hexahydro-1-ethyl-3a-methyl-indeno[2,1,b]pyrrol-5-ol in dry ether (or THF or benzene) was treated with 0.1 equivalent of NaOH and stirred for 30 minutes and added 1.1 equivalent or alkylisocynate at room temperature and stirred for 2 hours. The mixture was quenched with H₂O and extracted with CHCL₃ to give the desired 5-carbamate which was purified through silica gel column chromatography. Following this procedure, the following compounds were prepared.

### A. (+/-)-1,2,3,3a,8,8a-Hexahydro-1-ethyl-3a-methyl-indeno[2,1-b]pyrrol-5-ol, heptyl carbamate ester:

(Oil) ¹H NMR (CDCl₃) δ 0.84 (m, 3H), 1.4-1.6 (m, 2H), 1.9-2.1 (2H), 2.3-2.5 (m, 2H), 2.78-3.16 (m, 5H), 3.2 (q, 2H), 5.0 (t, 1H, NH), 6.8-6.9 (m, 2H), 7.06 (d, 1H) ppm.

### B. (-)-1,2,3,3a,8,8a-Hexahydro-1-ethyl-3a-methylindeno[2,1-b]pyrrol 5-ol, heptyl carbamate ester:

Anal. (C₂₂H₃₄N₂O₂.di-p-toluoyl-L-tartaric acid) calc. C, 67.72; H, 7.04; N, 3.76. Found: C, 67.77, H, 7.22; N, 4.00. [α]_{D} = -75.69°, mp 89-92°C.

### C. (+)-1,2,3,3a,8,8a-Hexahydro-1-ethyl-3a-methylindeno[2,1-b]pyrrol-5-ol, n-heptyl carbamate ester:

Anal. calc.: (C₂₂H₃₄N₂O₂.C₂₀H₁₈O₈) C, 67.72; H, 7.04; N, 3.76. Found: C, 67.14; H, 6.96; N, 3.82.

### D. (+/-)-1,2,3,3a,8,8a-Hexahydro-1-ethyl-3a-methylindeno[2,1-b]pyrrol-5-ol, n-hexyl carbamate ester:

(Oil) ¹H NMR (CDCl₃) δ 0.86 (m, 3H), 1.08 (t, 3H), 1.2-1.4 (m, 9H), 1.4-1.6 (m, 2H), 1.85-2.15 (m, 2H), 2.2-2.4 (m, 2H), 2.7-3.16 (m, 5H), 3.2 (q, 2H), 5.1 (t, 1H, NH), 6.8-7.0 (m, 2H), 7.08 (d, 1H) ppm.

### E. (-)-1,2,3,3a,8,8a-Hexahydro-1-ethyl-3a-methylindeno[2,1-b]pyrrol-5-ol, n-hexyl carbamate ester (from (-)-1,2,3,3a,8,8a-hexahydro-1-ethyl-3a-methyl-indeno-[2,1,b]pyrrol-5-ol and n-hexylisocyanate and sodium hydride):

[α]_{D} = -73.54°.

### F. (+)-1,2,3,3a,8,8a-Hexahydro-1-ethyl-3a-methyl-indeno[2,1-b]pyrrol-5-ol, n-hexyl carbamate ester:

[α]_{D} = +78.5°, the corresponding di-p-toluoyl-L-tartaric acid salt, mp 122-123.

### G. (+/-)-1,2,3,3a,8,8a-Hexahydro-1-ethyl-3a-methyl-indeno[2,1-b]pyrrol-5-ol, n-butyl carbamate ester:

(Oil) ¹H NMR (CDCl₃) δ 0.90 (t, 3H), 1.01 (t, 3H), 1.28 (s, 3H), 1.2-1.6 (m, 4H), 1.8-2.1 (m, 2H), 2.1-2.3 (m, 2H), 2.6-2.9 (m, 3H), 12.9-3.1 (m, 2H), 3.19 (q, 2H), 5.08 (t, 1H, NH), 6.7-6.9 (m, 2H), 7.0 (d, 1H) ppm.

### H. (+/-)-1,2,3,3a,8,8a-Hexahydro-1-ethyl-3a-methyl-indeno[2,1-b]pyrrol-5-ol, t-butyl carbamate ester:

(White crystals) ¹H NMR (CDCl₃) δ 1.07 (t, 3H), 1.28 (s, 3H), 1.32 (s, 9H), 1.8-2.1 (m, 2H), 2.2-2.4 (m, 2H), 2.7-3.1 (m, 5H), 4.88 brs, 1H), 6.8-6.9 (m, 2H), 7.04 (d, 1H) ppm. The tosylate salt: ¹H NMR (DMS₂O) δ 1.3-1.5 (m, 12H), 1.44 (s, 3H), 2.3-2.4 (m, 2H), 2.38 (s, 3H), 3.1-3.35 (m, 2H), 3.4-3.6 (m, 3H), 3.91 (d, 1H), 6.97 (m, 1H), 7.03 (s, 1H), 7.27 (d, 1H), 7.36 (d, 2H), 7.64 (d, 2H) ppm.

### I. (+/-)-1,2,3,3a,8,8a-Hexahydro-1-ethyl-3a-methyl-indeno[2,1-b]pyrrol-5-ol, n-propyl carbamate ester:

(Oil) ¹H NMR (CDCl₃) δ 0.94 (t, 3H), 1.14 (t, 3H), 1.32 (s, 3H), 1.5-1.7 (m, 2H), 1.9-2.2 (m, 2H), 2.3-2.5 (m, 2H), 2.7-3.2 (m, 5H), 3.2 (q, 2H), 5.0 (brs, 1H), 6.8-6.9 (m, 2H), 7.09 (d, 1H) ppm.

### J. (+/-)-1,2,3,3a,8,8a-Hexahydro-1-ethyl-3a-methyl-indeno[2,1-b]pyrrol-5-ol, methyl carbamate ester:

(White crystals) ¹H NMR (CDCl₃) δ 1.11 (t, 3H), 1.3 (s, 3H), 1.9-2.1 (m, 2H), 2.2-2.4 (m, 2H), 2.7-3.1 (m, 8H), 4.9 (brs, 1H), 6.8-6.9 (m, 2H), 7.08 (d, 1H) ppm.

### K. (+/-)-1,2,3,3a,8,8a-Hexahydro-1-ethyl-3a-methyl-indeno[2,1-b]pyrrol-5-ol, phenyl carbamate ester:

¹H NMR (CDCl₃) δ 1.22 (t, 3H), 1.42 (s, 3H), 2.0-1.5 (m, 4H), 2.8-3.3 (m, 5H), 7.0-7.3 (m, 4H), 7.4-7.6 (m, 4H) ppm.

### L. (+/-)-1,2,3,3a,8,8a-Hexahydro-1-ethyl-3a-methyl-indeno[2,1-b]pyrrol-5-ol, benzyl carbamate ester:

¹H NMR (CDCl₃) δ 1.2 (t, 3H), 1.4 (s, 3H), 1.9-2.2 (m, 2H), 2.3-2.5 (m, 2H), 2.8-3.2 (m, 5H), 4.45 (d, 2H), 5.48 (t, 1H, NH), 6.9 (m, 2H), 7.15 (d, 1H), 7.3-7.5 (m, 5H) ppm.

### M. (+/-)-1,2,3,3a,8,8a-Hexahydro-1-ethyl-3a-methyl-indeno[2,1-b]pyrrol-5-ol, (S)-α-methylbenzyl carbamate ester:

¹H NMR (CDCl₃) δ 1.12 (t, 3H), 1.3 (s, 3H), 1.56 (d, 3H), 1.8-2.1 (m, 2H), 2.2-2.4 (m, 2H), 2.7-3.1 (m, 5H), 4.9 (q, 1H), 5.245 (brs, 1H), 6.9 (m, 2H) , 7.05 (d, 1H), 7.24-7.4 (m, 5H)) ppm.

### N. (+/-)-1,2,3,3a,8,8a-Hexahydro-1-ethyl-3a-methyl-indeno[2,1-b]pyrrol-5-ol, n-heptyl carbamate ester tosylate salt:

(Oil) ¹H NMR (CDCl₃) δ 0.85 (t, 3H), 1.2-1.4 (m, 8H), 1.38 (s, 3H), 1.4-1.6 (m, 2H), 2.1-2.2 (m, 2H), 2.3 (s, 3H), 7.0-7.1 (m, 3H), 7.54 (d, 2H) ppm.

### O. (+/-)-1,2,3,3a,8,8a-Hexahydro-1,3a-dimethyl-indeno[2,1-b]pyrrol-5-ol, n-hexyl carbamate ester:

¹H NMR (CDCl₃) δ 0.86 (m, 3H), 1.1-1.6 (m, 11H), 1.8-2.6 (m, 6H), 2.7-3.3 (m, 6H), 5.1 (t, 1H, NH), 6.8-6.9 (m, 2H), 7.05 (d, 1H), ppm.

### P. (+/-)-1,2,3,3a,8,8a-Hexahydro-1-n-propyl-3a-indeno[2,1-b]pyrrol-5-ol, n-heptyl carbamate ester:

¹H NMR (CDCl₃) δ 0.8-1.0 (m, 6H), 1.0-1.6 (m, 15H), 1.8-2.4 (m, 4H), 2.5-3.3 (m, 7H), 5.18 (t, 1H, NH), 6.8 (m, 2H), 7.04 (d, 1H) ppm.

### Q. (+/-)-1,2,3,3a,8,8a-Hexahydro-1-n-propyl-3a-methyl-indeno[2,1-b]pyrrol-5-ol, n-hexyl carbamate ester:

¹H NMR (CDCl₃) δ 0.8 (m, 6H), 1.1-1.6 (m, 13H), 1.8-2.5 (m, 4H), 2.5-3.2 (m, 7H), 5.05 (t, 1H, NH), 6.7-6.8 (m, 2H), 7.0 (d, 1H), ppm.

### R. (+/-)-1,2,3,3a,8,8a-Hexahydro-1-benzyl-3a-methyl-indeno[2,1-b]pyrrol-5-ol, methyl carbamate ester:

(White crystals): ¹H NMR (CDCl₃) δ 1.31 (s, 3H), 1.8-2.0 (m, 2H), 2.3-2.4 (m, 1H), 2.7-3.0 (m, 5H), 3.25-3.4 (m, 3H), 3.95 (d, 1H), 6.8 (m, 2H), 7.14 (d, 1H), 7.2-7.3 (m, 5H), 8.4 (brs, 1H) ppm.

### S. (+/-)-1,2,3,3a,8,8a-Hexahydro-1-benzyl-3a-methyl-indeno[2,1-b]pyrrol-5-ol, n-hexyl carbamate ester:

¹H NMR (CDCl₃) δ 0.86 (t, 3H), 1.1-1.6 (m, 13H), 1.8-2.05 (m, 1H), 2.2-2.49 (m, 1H), 2.7-3.5 (m, 6H), 3.9 (d, 2H), 4.99 (t, 1H), 5.3 (t, 1H), 6.8-7.1 (m, 3H), 7.1-7.3 (m, 5H) ppm.

### T. (+/-)-1,2,3,3a,8,8a-Hexahydro-1-propyl-indeno-[2,1-b]pyrrol-5-ol, n-heptyl carbamate ester:

(White crystals) ¹H NMR (CDCl₃) δ 0.8-1.0 (m, 6H), 1.2-1.4 (m, 8H), 1.44-1.7 (m, 5H), 1.8-1.96 (m, 1H), 2.2-2.5 (m, 3H), 2.66-2.8 (m, 1H), 3.0-3.4 (m, 5H), 3.7-3.8 (m, 1H), 4.9-5.0 (m, 1H), 6.8-6.96 (m, 2H), 7.1 (d, 1H) ppm. C₂₂H₃₄N₂O₂ Calc.: C, 73.70; H, 9.56; N, 9.82. Found: C, 73.38; H, 9.69; N, 8.07.

### U. (+/-)-1,2,3,3a,8,8a-Hexahydro-1-propyl-indeno-[2,1-b]pyrrol-5-ol, n-hexyl carbamate ester:

(White crystals) ¹H NMR (CDCl₃) δ 0.8-1.0 (m, 6H), 1.2-1.4 (m, 6H), 1.4-1.6 (m, 4H), 1.7-1.9 (m, 1H), 2.15-2.4 (m, 3H), 2.6-2.8 (m, 1H), 2.8-3.3 (m, 6H), 3.6-3.8 m, 1H), 4.9-5.0 (m, 1H), 6.8-6.95 (m, 2H), 7.1 (d, 1H) ppm. C₂₁H₃₂N₂O₂ Calc.: C, 73.21; H, 9.36; N, 8.13. Found: C, 72.90; H, 9.80; N, 8.49.

### V. (+/-)-1,2,3,3a,8,8a-Hexahydro-1-n-propyl-indeno-[2,1-b]pyrrol-5-ol, n-hexyl carbamate ester:

[α]_{D} (CH₂Cl₂) = -130°.

### W. (+/-)-1,2,3,3a,8,8a-Hexahydro-1-methyl-indeno-[2,1-b]pyrrol-5-ol, heptyl carbamate ester:

(White crystals) ¹H NMR (CDCl₃) δ 0.88 (t, 3H), 1.2-1.4 (m, 8H), 1.46-1.6 (m, 2H), 1.8-2.0 (m, 1H), 2.2-2.4 (m, 2H), 2.38 (s, 3H), 2.8-3.04 (m, 4H), 3.16-3.3 (m, 2H), 3.64-3.8 (m, 1H), 4.9-5.0 (m, 1H), 6.84 (d, 1H), 6.9 (s, 1H), 7.12 (d, 1H) ppm. C₂₀H₃₀N₂O₂ Calc.: C, 72.69; H, 9.15; N, 8.48. Found: C, 72.45; H, 9.28; N. 8.53.

### X. (+/-)-1,2,3,3a,8,8a-Hexahydro-1-methyl-indeno-[2,1-b]pyrrol-5-ol, hexyl carbamate ester:

(White crystals) ¹H NMR (CDCl₃) δ 0.8 (t, 3H), 1.2-1.4 (m, 6H), 1.45-1.6 (m, 2H), 1.8-2.0 (m, 1H), 2.3-2.45 (m, 2H), 2.4 (s, 3H), 2.9-3.15 (m, 4H) , 3.15-3.3 (m, 2H), 3.65-3.8 (m, 1H), 6.85 (d, 1H), 6.9 (s, 1H), 7.1 (d, 1H) ppm. C₁₉H₂₈N₂O₂ Calc.: C, 72.11; H, 8.92; N, 8.86. Found: C, 71.46; H, 8.79; N, 8.66.

### Y. (+/-)-1,2,3,3a,8,8a-Hexahydro-1-ethyl-indeno-[2,1-b]pyrrol-5-ol, heptyl carbamate ester:

(White crystals) ¹H NMR (CDCl₃) δ 0.8-0.9 (m, 3H), 1.0-1.38 (m, 11H), 1.4-1.6 (m, 3H), 1.7-1.9 (m, 1H), 2.2-2.5 (m, 2H), 2.8-3.4 (m, 7H), 3.66-3.82 (m, 1H), 4.85-5.0 (m, 1H), 6.82 (d, 1H), 6.9 (s, 1H), 7.08 (d, 1H) ppm.

### Z. (+/-)-1,2,3,3a,8,8a-Hexahydro-1-ethyl-indeno-[2,1-b]pyrrol-5-ol, propyl carbamate ester:

(White crystals) ¹H NMR (CDCl₃) δ 0.8-1.0 (m, 3H), 1.12 (t, 3H), 1.26-1.9 (m, 4H), 2.2-2.4 (m, 3H), 2.75-3.3 (m, 6H), 3.6-3.8 (m, 1H), 4.9 (brs, 1H), 6.82 (d, 1H), 6.9 (s, 1H), 7.08 (d, 1H) ppm.

### AA. (+/-)-1,2,3,3a,8,8a-Hexahydro-1-ethyl-indeno-[2,1-b]pyrrol-5-ol, butyl carbamate ester:

(White crystals) ¹H NMR (CDCl₃) δ 0.8-1.0 (m, 3H), 1.0-1.6(m, 8H), 1.7-1.9 (m, 1H), 2.2-2.6 (m, 3H), 2.75-3.4 (m, 6H), 3.6-3.8 (m, 1H), 4.9 (brs, 1H), 6.82 (d, 1H), 6.88 (s, 1H), 7.07 (d, 1H) ppm.

### AB. (+/-)-1,2,3,3a,8,8a-Hexahydro-1-ethyl-indeno-[2,1-b]pyrrol-5-ol, hexyl carbamate ester:

(White crystals) ¹H NMR (CDCl₃) δ 0.8-1.0 (m, 3H), 1.1-1.6 (m, 11H), 1.7-1.9 (m, 1H), 2.4-2.6 (m, 3H), 2.75-3.3 (m, 7H), 3.6-3.8 (m, 1H), 4.94 (brs, 1H), 6.82 (d, 1H), 6.90 (s, 1H), 7.08 (de, 1H) ppm.

The activity of the compounds of the present invention as cholinesterase inhibitors and analgesic agents may be determined by a number of standard biological or pharmacological tests. One such procedure for determining cholinesterase inhibition is described by Ellman et al. in "A New and Rapid Colorimetric Determination of Acetylchlinesterase Activity", Biochem. Pharm. 1, 88, (1961). Examples of such procedures for determining analgesic activity are the hot plate assay described in Lab. Animal, 7, 42 (1978), and the tail-flick and phenylquinone assays described in J. Pharmacol. Exp. Ther., 175, 435 (1970) and J. Pharmacol. Exp. Ther., 179, 652 (1971).

The title compounds of Example 9 were tested for AChE inhibition assay as described in the above procedure. All of the compounds were effective at a level of 1 µM to 10 µM or IC₅₀.

Human AChE (EC 3.1.1.7) purified from red blood cells and butyrylcholine iodide esterase (BuChE; EC 3.1.1.8) purified from serum were purchased from Sigma Chemical Company (St. Louis, MO). Assays were performed as described by Ellman et al., (19061), using 200 µM acetylthiocholine iodide as substrate for AChE and 500 µM butyrylthiocholine iodide as substrate for BuChE. Kinetic parameters were calculated from enzyme inhibition curves fitted with non-linear least squares using the programs COMP, UCOMP and NCOMP (Cleland, 1979).

## Claims (Claims for the following Contracting State(s): AT, BE, CH, DE, DK, FR, GB, IT, LI, LU, NL, SE)

1. A compound of the formula 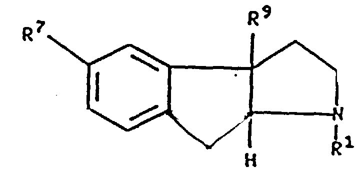 wherein
R¹ is (C₁ to C₈)alkyl or phenyl (C₁ to C₄)alkyl;
R⁷ is OCONR¹³H or OCSNR¹³H wherein R¹³ is (C₁ to C₁₂)alkyl, phenyl or phenyl (C₁-C₈)alkyl; and
R⁹ is hydrogen or (C₁ to C₈)alkyl;
and pharmaceutically acceptable acid addition salts thereof.

2. A compound according to claim 1 wherein R¹ is methyl, ethyl, propyl or benzyl; R⁷ is OCONR¹³H wherein R¹³ is (C₁ to C₁₂)alkyl, phenyl or benzyl; and R⁹ is hydrogen or methyl.

3. A compound according to claim 2 wherein R¹³ is (C₁ to C₇)alkyl and R⁹ is methyl.

4. A compound according to claim 1 wherein R⁷ is OCONR¹³H wherein R¹³ is n-butyl, n-hexyl or n-heptyl; and R⁹ is hydrogen or methyl; in the form of the (-) enantiomer.

5. A pharmaceutical composition comprising a compound according to any one of claims 1 to 4, or a pharmaceutically acceptable acid addition salt thereof, in combination with a pharmaceutically acceptable carrier.

6. A compound according to any one of claims 1 to 4, or a pharmaceutically acceptable acid addition salt thereof, or a pharmaceutical composition according to claim 5, for use as a medicament.

7. The use of a compound according to any one of claims 1 to 4, or a pharmaceutically acceptable acid addition salt thereof, or a pharmaceutical composition according to claim 5, for the manufacture of a medicament for the treatment of memory dysfunctions characterised by decreased cholinergic function such as Alzheimer's disease.

8. A compound of the formula 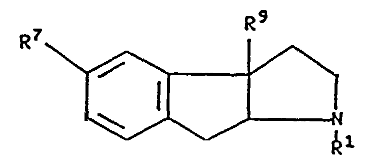 wherein R⁷ is O(C₁ to C₄)alkyl; R⁹ is (C₁ to C₄)alkyl; and R¹ is as defined in claim 1; provided that when R⁷ is methoxy and R⁹ is methyl, then R¹ is not hydrogen, methyl, n-propyl, n-pentyl, benzyl or 2-phenylethyl; and pharmaceutically acceptable acid addition salts thereof.

9. A compound of the formula 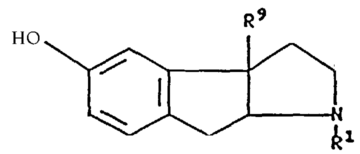 wherein R⁹ is as defined in claim 8; and R¹ is as defined in claim 1; provided that when R⁹ is methyl, then R¹ is not hydrogen or methyl; and pharmaceutically acceptable acid addition salts thereof.

## Claims (Claims for the following Contracting State(s): ES)

1. A process for the preparation of a compound of the formula 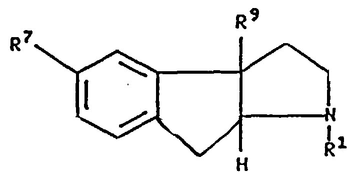 wherein
R¹ is (C₁ to C₄)alkyl or phenyl (C₁ to C₄)alkyl;
R⁷ is OCONR¹³H or OCSNR¹³H wherein R¹³ is (C₁ to C₁₂)alkyl, phenyl or phenyl (C₁-C₄)alkyl; and
R⁹ is (C₁ to C₄)alkyl;
and pharmaceutically acceptable acid addition salts thereof;
which comprises reaction of a compound of the formula 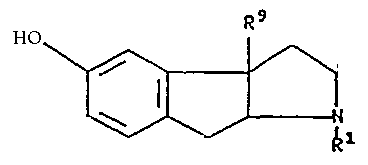 wherein R¹ and R⁹ are as previously defined in this claim with,
(a) the appropriate isocyanate or isothiocyanate of formula R¹³N=C=O or R¹³N=C=S respectively, in the presence of a catalytic amount of a base in an aprotic solvent at from 0 to 50°C, or
(b) 1,1'-carbonyldiimidazole or 1,1'-thiocarbonyldiimidazole, followed by the appropriate amine of formula R¹³NH₂ wherein R¹³ is as previously defined in this claim, in an aprotic solvent at from -40 to 100°C;
followed in each case by optional formation and isolation of a pharmaceutically acceptable acid addition salt of the required product.

2. A process according to claim 1 wherein in
(a) the base is sodium hydride, sodium, or sodium alkoxide, the solvent is tetrahydrofuran, ether, benzene or toluene, and the reaction temperature is 20°C, and in
(b) the solvent is methylene chloride, tetrahydrofuran or toluene, and the reaction temperature is from 20 to 70°C.

## Claims (Claims for the following Contracting State(s): GR)

1. A process for the preparation of a compound of the formula 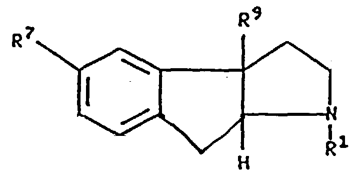 wherein
R¹ is (C₁ to C₄)alkyl or phenyl (C₁ to C₄)alkyl;
R⁷ is OCONR¹³H or OCSNR¹³H wherein R¹³ is (C₁ to C₁₂)alkyl, phenyl or phenyl (C₁-C₄)alkyl; and
R⁹ is (C₁ to C₄)alkyl;
and pharmaceutically acceptable acid addition salts thereof;
which comprises reaction of a compound of the formula 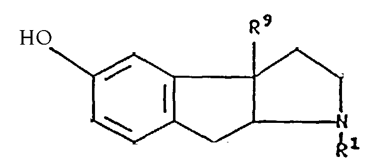 wherein R¹ and R⁹ are as previously defined in this claim with,
(a) the appropriate isocyanate or isothiocyanate of formula R¹³N=C=O or R¹³N=C=S respectively, in the presence of a catalytic amount of a base in an aprotic solvent at from 0 to 50°C, or
(b) 1,1'-carbonyldiimidazole or 1,1'-thiocarbonyldiimidazole, followed by the appropriate amine of formula R¹³NH₂ wherein R¹³ is as previously defined in this claim, in an aprotic solvent at from -40 to 100°C;
followed in each case by optional formation and isolation of a pharmaceutically acceptable acid addition salt of the required product.

2. A process according to claim 1 wherein in
(a) the base is sodium hydride, sodium, or sodium alkoxide, the solvent is tetrahydrofuran, ether, benzene or toluene, and the reaction temperature is 20°C, and in
(b) the solvent is methylene chloride, tetrahydrofuran or toluene, and the reaction temperature is from 20 to 70°C.

3. A compound of the formula 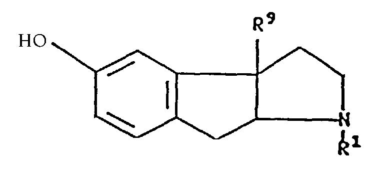 wherein R¹ and R⁹ are as defined in claim 1; provided that when R⁹ is methyl, then R¹ is not hydrogen or methyl; and pharmaceutically acceptable acid addition salts thereof.

## Patentansprüche (Patentansprüche für folgende(n) Vertragsstaat(en): AT, BE, CH, DE, DK, FR, GB, IT, LI, LU, NL, SE)

1. Verbindung der Formel 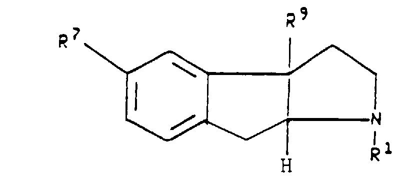 worin
R¹ (C₁ bis C₈)-Alkyl oder Phenyl (C₁ bis C₄)alkyl darstellt;
R⁷ OCONR¹³H oder OCSNR¹³H darstellt, wobei R¹³ (C₁ bis C₁₂)-Alkyl, Phenyl oder Phenyl(C₁-C₈)alkyl darstellt; und
R⁹ Wasserstoff oder (C₁ bis C₈)-Alkyl darstellt
und pharmazeutisch verträgliche Säureadditionssalze davon.

2. Verbindung nach Anspruch 1, worin R¹ Methyl, Ethyl, Propyl oder Benzyl darstellt; R⁷ OCONR¹³H darstellt, wobei R¹³ (C₁ bis C₁₂)-Alkyl, Phenyl oder Benzyl darstellt und R⁹ Wasserstoff oder Methyl darstellt.

3. Verbindung nach Anspruch 2, worin R¹³ (C₁ bis C₇)-Alkyl darstellt und R⁹ Methyl darstellt.

4. Verbindung nach Anspruch 1, worin R⁷ OCONR¹³H darstellt, wobei R¹³ n-Butyl, n-Hexyl oder n-Heptyl darstellt; und R⁹ Wasserstoff oder Methyl darstellt; in Form des (-)Enantiomers.

5. Pharmazeutische Zusammensetzung, umfassend eine Verbindung nach einem der Ansprüche 1 bis 4 oder ein pharmazeutisch verträgliches Säureadditionssalz davon, in Kombination mit einem pharmazeutisch verträglichen Träger.

6. Verbindung nach einem der Ansprüche 1 bis 4 oder ein pharmazeutisch verträgliches Säureadditionssalz davon oder eine pharmazeutische Zusammensetzung nach Anspruch 5 zur Verwendung als Arzneimittel.

7. Verwendung einer Verbindung nach einem der Ansprüche 1 bis 4 oder eines pharmazeutisch verträglichen Säureadditionssalzes davon oder einer pharmazeutischen Zusammensetzung nach Anspruch 5, zur Herstellung eines Arzneimittels für die Behandlung von Gedächtnisstörungen, die durch verminderte cholinerge Funktion gekennzeichnet sind, wie Alzheimer-Krankheit.

8. Verbindung der Formel 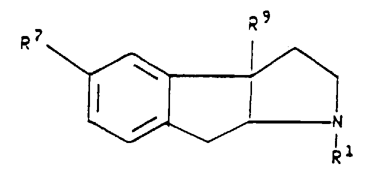 worin R⁷ O(C₁ bis C₄)-Alkyl darstellt; R⁹ (C₁ bis C₄)-Alkyl darstellt und R¹ wie in Anspruch 1 definiert ist, mit der Maßgabe, daß, wenn R⁷ Methoxy darstellt und R⁹ Methyl darstellt, R¹ dann nicht Wasserstoff, Methyl, n-Propyl, n-Pentyl, Benzyl oder 2-Phenylethyl darstellt und pharmazeutisch verträgliche Säureadditionssalze davon.

9. Verbindung der Formel 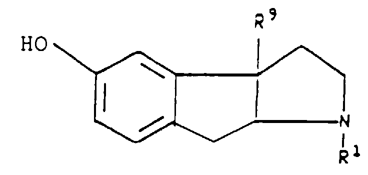 worin R⁹ wie in Anspruch 8 definiert ist und R¹ wie in Anspruch 1 definiert ist, mit der Maßgabe, daß, wenn R⁹ Methyl darstellt, R¹ dann nicht Wasserstoff oder Methyl darstellt und pharmazeutisch verträgliche Säureadditionssalze davon.

## Patentansprüche (Patentansprüche für folgende(n) Vertragsstaat(en): ES)

1. Verfahren zur Herstellung einer Verbindung der Formel 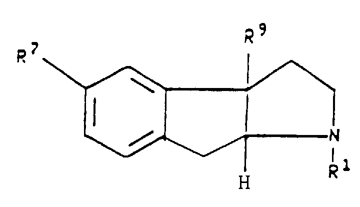 worin
R¹ (C₁ bis C₄)-Alkyl oder Phenyl(C₁ bis C₄)-alkyl darstellt;
R⁷ OCONR¹³H oder OCSNR¹³H darstellt, worin R¹³ (C₁ bis C₁₂)-Alkyl, Phenyl oder Phenyl(C₁-C₄)alkyl darstellt; und
R⁹ (C₁ bis C₄)-Alkyl darstellt
und pharmazeutisch verträglicher Säureadditionssalze davon;
umfassend Umsetzung einer Verbindung der Formel 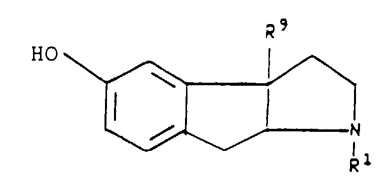 worin R¹ und R⁹ wie vorstehend in diesem Anspruch definiert sind, mit
(a), dem geeigneten Isocyanat oder Isothiocyanat der Formel R¹³N=C=O bzw. R¹³N=C=S, in Gegenwart einer katalytischen Menge einer Base in einem aprotischen Lösungsmittel bei 0 bis 50°C oder
(b) 1,1'-Carbonyldiimidazol oder 1,1'-Thiocarbonyldiimidazol, gefolgt von dem geeigneten Amin der Formel R¹³NH₂, worin R¹³ wie vorstehend in diesem Anspruch definiert ist, in einem aprotischen Lösungsmittel bei -40 bis 100°C;
in jedem Fall gegebenenfalls gefolgt von Bildung und Gewinnung eines pharmazeutisch verträglichen Säureadditionssalzes des gewünschten Produkts.

2. Verfahren nach Anspruch 1, wobei in
(a) die Base Natriumhydrid, Natrium oder Natriumalkoxid darstellt, das Lösungsmittel Tetrahydrofuran, Ether, Benzol oder Toluol darstellt und die Reaktionstemperatur 20°C ist und in
(b) das Lösungsmittel Methylenchlorid, Tetrahydrofuran oder Toluol darstellt und die Reaktionstemperatur 20 bis 70°C ist.

## Patentansprüche (Patentansprüche für folgende(n) Vertragsstaat(en): GR)

1. Verfahren zur Herstellung einer Verbindung der Formel 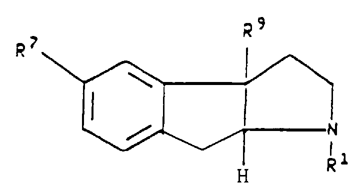 worin
R¹ (C₁ bis C₄)-Alkyl oder Phenyl(C₁ bis C₄)alkyl darstellt;
R⁷ OCONR¹³H oder OCSNR¹³H darstellt, worin R¹³ (C₁ bis C₁₂)-Alkyl, Phenyl oder Phenyl(C₁-C₄)alkyl darstellt; und
R⁹ (C₁ bis C₄)-Alkyl darstellt
und pharmazeutisch verträglicher Säureadditionssalze davon;
umfassend Umsetzung einer Verbindung der Formel 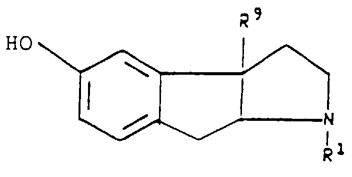 worin R¹ und R⁹ wie vorstehend in diesem Anspruch definiert sind, mit
(a), dem geeigneten Isocyanat oder Isothiocyanat der Formel R¹³N=C=O bzw. R¹³N=C=S, in Gegenwart einer katalytischen Menge einer Base in einem aprotischen Lösungsmittel bei 0 bis 50°C oder
(b) 1,1'-Carbonyldiimidazol oder 1,1'-Thiocarbonyldiimidazol, gefolgt von dem geeigneten Amin der Formel R¹³NH₂, worin R¹³ wie vorstehend in diesem Anspruch definiert ist, in einem aprotischen Lösungsmittel bei -40 bis 100°C;
in jedem Fall gegebenenfalls gefolgt von Bildung und Gewinnung eines pharmazeutisch verträglichen Säureadditionssalzes des gewünschten Produkts.

2. Verfahren nach Anspruch 1, wobei in
(a) die Base Natriumhydrid, Natrium oder Natriumalkoxid darstellt, das Lösungsmittel Tetrahydrofuran, Ether, Benzol oder Toluol darstellt und die Reaktionstemperatur 20°C ist und in
(b) das Lösungsmittel Methylenchlorid, Tetrahydrofuran oder Toluol darstellt und die Reaktionstemperatur 20 bis 70°C ist.

3. Verbindung der Formel 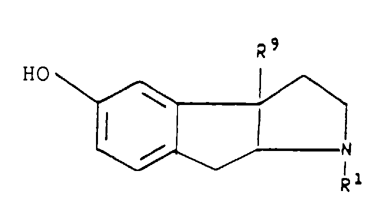 worin R¹ und R⁹ wie in Anspruch 1 definiert sind, mit der Maßgabe, daß, wenn R⁹ Methyl darstellt, R¹ dann nicht Wasserstoff oder Methyl darstellt und pharmazeutisch verträgliche Säureadditionssalze davon.

## Revendications (Revendications pour l'(les) Etat(s) contractant(s) suivant(s): AT, BE, CH, DE, DK, FR, GB, IT, LI, LU, NL, SE)

1. Composé de formule : 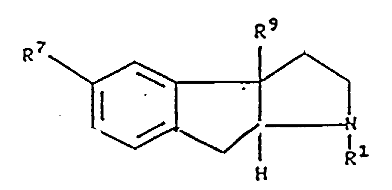 dans laquelle :
R¹ représente un radical alkyle en C₁ à C₈ ou phényl(alkyle en C₁ à C₄);
R⁷ représente OCONR¹³H ou OCSNR¹³H dans lequel R¹³ représente un radical alkyle en C₁ à C₁₂, phényle, ou phényl(alkyle en C₁ à C₈); et
R⁹ représente un atome d'hydrogène ou un radical alkyle en C₁ à C₈;
et ses sels d'addition d'acides pharmaceutiquement acceptables.

2. Composé selon la revendication 1 dans lequel R¹ est un radical méthyle, éthyle, propyle ou benzyle; R⁷ représente OCONR¹³H dans lequel R¹³ représente un radical alkyle en C₁ à C₁₂, phényle ou benzyle; et R⁹ représente un atome d'hydrogène ou un radical méthyle.

3. Composé selon la revendication 2 dans lequel R¹³ représente un radical alkyle en C₁ à C₇ et R⁹ représente un radical méthyle.

4. Composé selon la revendication 1 dans lequel R⁷ représente OCONR¹³H dans lequel R¹³ représente un radical n-butyle, n-hexyle ou n-heptyle; et R⁹ représente un atome d'hydrogène ou un radical méthyle; sous la forme de l'énantiomère (-).

5. Composition pharmaceutique comprenant un composé selon l'une quelconque des revendications 1 à 4, ou son sel d'addition d'acide pharmaceutiquement acceptable, en association avec un vecteur pharmaceutiquement acceptable.

6. Composé selon une quelconque des revendications 1 à 4, ou son sel d'addition d'acide pharmaceutiquement acceptable, ou composition pharmaceutique selon la revendication 5, pour utilisation comme médicament.

7. Utilisation d'un composé selon l'une quelconque des revendications 1 à 4, ou son sel d'addition d'acide pharmaceutiquement acceptable, ou d'une composition pharmaceutique selon la revendication 5, pour la fabrication d'un médicament pour le traitement des troubles de la mémoire caractérisés par une fonction cholinergique diminuée tels que la maladie d'Alzheimer.

8. Composé de formule : 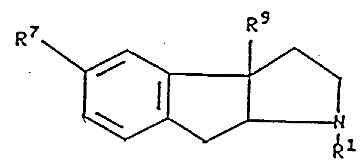 dans laquelle R⁷ représente un radical O-(alkyle en C₁ à C₄); R⁹ représente un radical alkyle en C₁ à C₄; et R¹ est tel que défini dans la revendication 1; à condition que lorsque R⁷ est un radical méthoxy et R⁹ est un radical méthyle, alors R¹ ne soit ni un atome d'hydrogène, ni un radical méthyle, n-propyle, n-pentyle, benzyle ou 2-phényléthyle; et ses sels d'addition d'acides pharmaceutiquement acceptables.

9. Composé de formule : 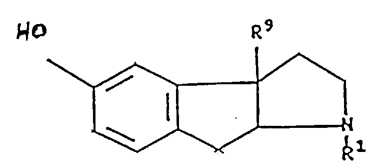 dans laquelle R⁹ est tel que défini dans la revendication 8; et R¹ est tel que défini dans la revendication 1; à condition que lorsque R⁹ est un radical méthyle, alors R¹ ne soit ni un atome d'hydrogène ni un radical méthyle; et ses sels d'addition d'acides pharmaceutiquement acceptables.

## Revendications (Revendications pour l'(les) Etat(s) contractant(s) suivant(s): ES)

1. Procédé pour la préparation d'un composé de formule : 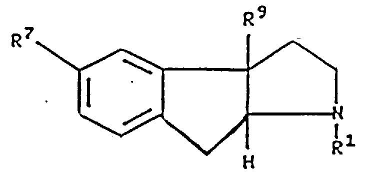 dans laquelle :
R¹ représente un radical alkyle en C₁ à C₄ ou phényl(alkyle en C₁ à C₄);
R⁷ représente OCONR¹³H ou OCSNR¹³H dans lequel R¹³ représente un radical alkyle en C₁ à C₁₂, phényle ou phényl(alkyle en C₁ à C₄); et
R⁹ représente un radical alkyle en C₁ à C₄;
et ses sels d'addition d'acides pharmaceutiquement acceptables;
qui comprend la réaction d'un composé de formule : 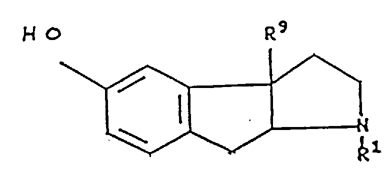 dans laquelle R¹ et R⁹ sont tels que définis précédemment dans cette revendication avec,
(a) l'isocyanate ou l'isothiocyanate approprié de formule R¹³N=C=O ou R¹³N=C=S respectivement, en présence d'une quantité catalytique d'une base dans un solvant aprotique entre 0 et 50°C, ou
(b) du 1,1'-carbonyldiimidazole ou du 1,1'-thiocarbonyldiimidazole, suivi par l'amine appropriée de formule R¹³NH₂ dans laquelle R¹³ est tel que précédemment défini dans cette revendication, dans un solvant aprotique entre -40 et 100°C;
suivie dans chaque cas par la formation et l'isolation éventuelles d'un sel d'addition d'acide pharmaceutiquement acceptable du produit souhaité.

2. Procédé selon la revendication 1 dans lequel en
(a) la base est l'hydrure de sodium, le sodium, ou un alcoolate de sodium, le solvant est le tétrahydrofuranne, l'éther, le benzène ou le toluène, et la température de réaction est 20°C, et en
(b) le solvant est le chlorure de méthylène, le tétrahydrofuranne ou le toluène, et la température de réaction est comprise entre 20 et 70°C.

## Revendications (Revendications pour l'(les) Etat(s) contractant(s) suivant(s): GR)

1. Procédé pour la préparation d'un composé de formule : 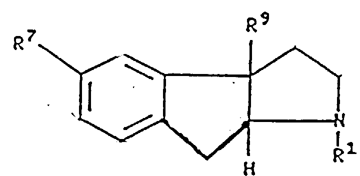 dans laquelle :
R¹ représente un radical alkyle en C₁ à C₄ ou phényl(alkyle en C₁ à C₄);
R⁷ représente OCONR¹³H ou OCSNR¹³H dans lequel R¹³ représente un radical alkyle en C₁ à C₁₂, phényle ou phényl(alkyle en C₁ à C₄); et
R⁹ représente un radical alkyle en C₁ à C₄;
et ses sels d'addition d'acides pharmaceutiquement acceptables;
qui comprend la réaction d'un composé de formule : 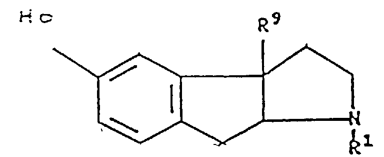 dans laquelle R¹ et R⁹ sont tels que définis précédemment dans cette revendication avec,
(a) l'isocyanate ou l'isothiocyanate approprié de formule R¹³N=C=O ou R¹³N=C=S respectivement, en présence d'une quantité catalytique d'une base dans un solvant aprotique entre 0 et 50°C, ou
(b) du 1,1'-carbonyldiimidazole ou du 1,1'-thiocarbonyldiimidazole, suivi par l'amine appropriée de formule R¹³NH₂ dans laquelle R¹³ est tel que précédemment défini dans cette revendication, dans un solvant aprotique entre -40 et 100°C;
suivie dans chaque cas par la formation et l'isolation éventuelles d'un sel d'addition d'acide pharmaceutiquement acceptable du produit souhaité.

2. Procédé selon la revendication 1 dans lequel en
(a) la base est l'hydrure de sodium, le sodium, ou un alcoolate de sodium, le solvant est le tétrahydrofuranne, l'éther, le benzène ou le toluène, et la température de réaction est 20°C, et en
(b) le solvant est le chlorure de méthylène, le tétrahydrofuranne ou le toluène, et la température de réaction est comprise entre 20 et 70°C.

3. Composé de formule : 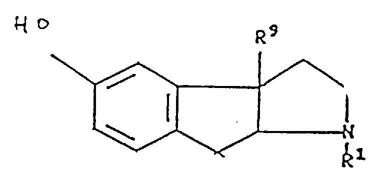 dans laquelle R¹ et R⁹ sont tels que définis dans la revendication 1; à condition que lorsque R⁹ représente un radical méthyle, alors R¹ ne soit ni un atome d'hydrogène ni un radical méthyle; et ses sels d'addition d'acides pharmaceutiquement acceptables.
